# EUROPÄISCHE PATENTANMELDUNG

(11) **EP 0 794 196 A2**
(43) Veröffentlichungstag der Anmeldung: **10.09.1997**
(21) Anmeldenummer: 97102723.0
(22) Anmeldetag: 20.02.1997
(51) Int. Cl.: C07K 16/18, G01N 33/577

(54) **Antikörper zur selektiven immunologischen Bestimmung von Gallensäuren in biologischen Matrices**

(30) Priorität: 06.03.1996 DE 19608592
(71) Anmelder: HOECHST AKTIENGESELLSCHAFT, 65929 Frankfurt am Main (DE)
(72) Erfinder: Müllner, Stefan, Dr., 65239 Hochheim (DE); Hoffmann, Axel, Dr., 65929 Frankfurt (DE)

(57) **Zusammenfassung**

Es werden polyklonale Antikörper zur selektiven immunologischen Bestimmung von Gallensäuren in biologischen Matrices, ein Verfahren zur Herstellung dieser Antikörper, sowie deren Verwendung in Immunoassays beschrieben.

## Beschreibung

Die vorliegende Erfindung betrifft polyklonale Antikörper zur selektiven immunologischen Bestimmung von Gallensäuren in biologischen Matrices, ein Verfahren zur Herstellung dieser Antikörper, sowie deren Verwendung in Immunoassays.

Gallensäuren bilden das Endprodukt des Cholesterin Stoffwechsels und ihre Bestimmung hat große diagnostische Bedeutung im Hinblick auf die Erkennung und Behandlung von Stoffwechselstörungen, Lebererkrankungen und Magen-Darm Fehlfunktionen (K.D.R. Setchell and A. Matsui, Clin. Chim. Acta 127, 1983, S. 1-17 ).

Die gegenwärtig zur Verfügung stehenden Verfahren zur exakten Bestimmung von Gallensäuren in biologischen Matrices, wie z.B. Serum, Feces, Urin und Gewebeproben haben den Nachteil, entweder sehr arbeitsaufwendig für die klinische Praxis zu sein, oder zu wenig selektiv bzw. zu unempfindlich zu sein. Immunoassays haben dagegen den entscheidenen Vorteil, nicht nur außerordentlich selektiv zu sein, sondern auch hochempfindliche Bestimmungen zu erlauben.

Die bisherigen Immunoassays für Gallensäuren waren auf Grund der spezifischen Eigenschaften des Analyten alle als kompetitive Radioimmunoassays ausgelegt, da die Art der Immunisierung (Verwendung von Rinderserumalbumin gekoppelte Gallensäuren) nur zu wenig spezifischen und schlecht bindenden Antikörper führte (A. Roda et al., Talanta 31, S. 895-900, 1984).

Demgegenüber ist es Aufgabe der vorliegenden Erfindung, polyklonale Antikörper, die gute Bindungseigenschaften und eine hohe Spezifität gegenüber Gallensäuren aufweisen und ein Verfahren zu deren Herstellung bereitzustellen.

Der vorliegenden Erfindung liegt ferner die Aufgabe zugrunde, ein Verfahren zur immunologischen Bestimmung von Gallensäuren auf Basis der zu entwickelnden Antikörper bereitzustellen, bei dem eine schnelle und genaue Bestimmung des Gallensäuregehalts in biologischen Matrices gewährleistet ist.

Die Aufgabe wird erfindungsgemäß gelöst durch einen polyklonalen Antikörper
1. mit Spezifität zu Gallensäure homopolymeren,
2. sowie mit Spezifität zu den jeweiligen repetitiven Untereinheiten.

Die gestellte Aufgabe wird darüber hinaus gelöst durch ein Verfahren zur immunologischen Bestimmung von Gallensäuren unter Verwendung eines Antikörpers und eines markierten Zweitantikörpers, der an ersteren Antikörper bindet, dadurch gekennzeichnet, daß der erste Antikörper ein polyclonaler Antikörper gemäß der vorliegenden Erfindung ist. Im folgenden wird die Erfindung detailliert erläutert. Ferner wird die Erfindung durch die Patentansprüche definiert.

Durch Immunisierung von einer geeigneten Wirbeltierart, wie z.B. Kaninchen, Schaf, Schwein, Ziege, Huhn etc. mit einem speziellen Homopolymer, ist es nun gelungen, Antikörper nicht nur gegen das Antigen selbst, sondern auch gegen die repetitiven Untereinheiten zu generieren. Dieses Verfahren stellt einen völlig neuen Weg dar, Antikörper gegen niedermolekulare Einheiten zu generieren.

Bei diesem speziellen Homopolymer handelt es sich um polymere oder oligomere Gallensäuren, herstellbar durch Polymerisation von monomeren Gallensäuren der Formel I

G - X - A (I)

in der
- G: ein Gallensäurerest oder -derivat,
- X: eine Brückengruppe und
- A: eine polymersierbare, ethylenisch ungesättigte Gruppe bedeutet.

Unter den Verbindungen der Formel I sind bevorzugt folgende:

Verbindungen, in denen
- G: eine freie Gallensäure bzw. ihr Alkali- oder Erdalkalisalz oder eine am Ring D veresterte Gallensäure, die über ihren Ring A oder B, vorzugsweise über Ring A, verbunden ist mit der Gruppe
- X: für die bevorzugt die Formel II gilt

(Y)ₒ - (Z)ₚ (II),

worin
- Y: benachbart zu G steht und -O-, -NR'-, -O-(C=O)-, -NR'-(C=O)-,
- Z: (C₁-C₁₂)-Alkylen oder (C₇-C₁₃)-Aralkylen, wobei einzelne, bevorzugt 1 bis 4, Methylengruppen in der Alkylenkette des Alkylen- oder Aralkylenrestes durch Gruppen wie -O-, -NR'-, -NR'-(C=O)-, -O-(C=O)- oder -NR'-(C=O)-NR''-, bevorzugt eine Gruppe eines Types, ersetzt sein können,
- o und p: unabhängig voneinander null oder 1, wobei o und p nicht gleichzeitig null sind,
- A: eine ethylenisch ungesättigte Gruppe der Formel 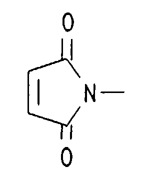 oder 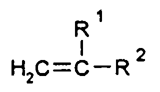 bedeuten, worin
- R¹: Wasserstoff oder CH₃ und
- R²: -NR'-(C=O)-, -O-(C=O)-, -O-, -NR'- oder eine Einfachbindung bedeutet, wobei die Carbonylgruppen benachbart zur C-C-Doppelbindung stehen,
- R': Wasserstoff oder (C₁-C₆)-Alkylen, bevorzugt (C₁-C₃)-Alkylen bedeuten.

Hierunter bevorzugt sind Polymere und Oligomere, worin G der Formel III 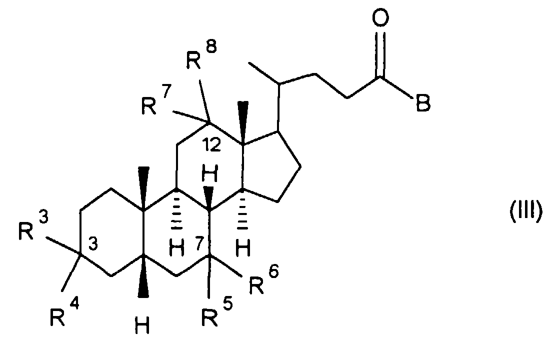 entspricht, worin
- R³ bis R⁸: unabhängig voneinander Wasserstoff, OH, NH₂ oder eine mit einer OH-Schutzgruppe geschützte OH-Gruppe und einer der Reste R³ bis R⁶ eine Bindung zur Gruppe X bedeuten, wobei diese Bindung von den Positionen 3 (R³ oder R⁴) oder 7 (R⁵ oder R⁶), bevorzugt ist die β-Position, ausgeht und die jeweils andere Position 7 oder 3 eine OH-Gruppe oder eine geschützte OH-Gruppe trägt,
- B: -OH, -O-Alkali, -O-Erdalkali, -O-(C₁₋C₁₂)-Alkyl, -O-Allyl oder -O-Benzyl bedeutet, bevorzugt -OH, -O-Alkali, -O-(C₁-C₆)-Alkyl, -O-Allyl oder -O-Benzyl, wobei Alkyl sowohl n-Alkyl wie iso-Alkyl bedeutet und wobei die gebildete Estergruppe 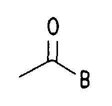 sowohl sauer wie auch basisch verseifbare Ester darstellt,
- Y: -O-, -NR'-, -O-(C=O)-, -NR'-(C=O)-,
- Z: (C₁-C₁₂)-Alkylen, (C₇-C₁₃)-Aralkylen, wobei 1 bis 3 Methylengruppen in der Alkylenkette durch die Gruppen -O-, -NR'-, -NR'-(C=O)-, -O-(C=O)-, -NR'-(C=O)-NR''-ersetzt sind und
- o und p: unabhängig voneinander null oder 1 bedeuten, wobei o und p nicht gleichzeitig null sind,
- A: 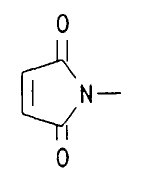 oder 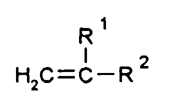 bedeuten, wobei
- R¹: Wasserstoff oder CH₃ und
- R²: -NR'-(C=O)-, -NR'- oder eine Einfachbindung bedeutet, worin
- R': Wasserstoff oder (C₁-C₆)-Alkylen bedeuten.

Sofern p = null und o = 1 gilt, ist Y bevorzugt -O-(C=O)- oder -NR'-(C=O)-.

Sofern p = 1 und o = null gilt, ist Z bevorzugt (C₁-C₁₂)-Alkylen, wobei 1 - 3 Methylengruppen, bevorzugt eine Methylengruppe, durch -NR'-(C=O)-NR'' ersetzt sind.
R' und R'' unabhängig voneinander Wasserstoff oder (C₁-C₆)-Alkylen bedeuten.

Sofern p = 1 und o = 1 gilt, ist Y bevorzugt -O-. Hierunter ist bevorzugt, daß Z (C₁-C₁₂)-Alkylen oder (C₇-C₁₃)-Aralkylen bedeutet, wobei 1 oder 2 Methylengruppen, bevorzugt eine Methylengruppe, durch -NR'-(C=O)- oder -NR'-(C=O)-NR''- ersetzt sind.
R' und R'' unabhängig voneinander Wasserstoff oder (C₁-C₆)-Alkylen bedeuten.

Weiterhin ist hierunter bevorzugt, daß eine Methylengruppe von Z dann -NR'-(C=O)-NR''- bedeutet, wenn Z selbst ein Aralkylrest bedeutet, worin der Arylrest meta-verknüpft ist, Z einerseits als Rest A eine Gruppe 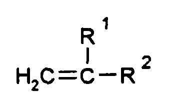 trägt, worin R² eine Einfachbindung bedeutet und andererseits eine -NR'-(C=O)-NR''- Gruppe trägt, die über eine Methylengruppe mit den Aralkylenrest meta-verknüpft ist.
R' und R'' unabhängig voneinander Wasserstoff oder (C₁-C₆)-Alkylen bedeuten.

Ebenso ist hierunter bevorzugt, daß, sofern Z eine (C₁-C₁₂)-Alkylengruppe bedeutet,
maximal eine Methylengruppe durch -NR'-(C=O)- ersetzt ist und als Rest A 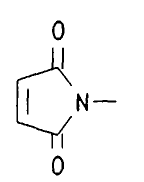 oder 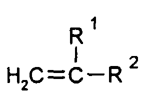 gilt, wobei R² -NR'-(C=O)-bedeutet.

Besonders bevorzugt ist weiterhin, daß Y nicht direkt mit der eine Methylengruppe von Z ersetzenden Gruppe direkt benachbart ist und auch nicht mit 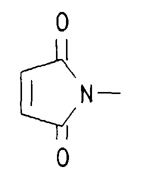 oder 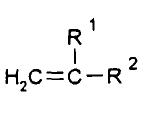 benachbart ist, sofern R² eine Einfachbindung bedeutet.

Unter den OH-Schutzgruppen werden verstanden
ein Alkyrest mit 1 - 10 C-Atomen oder ein Alkenylrest mit 2 - 10 C-Atomen, wobei die Reste verzweigt oder unverzweigt sind,
ein Cycloalkylrest mit 3-8 C-Atomen,
ein Phenylrest, der unsubstituiert oder 1-3fach substituiert ist mit F, Cl, Br, (C₁-C₄)-Alkyl oder (C₁-C₄)-Alkoxy,
ein Benzylrest, der unsubstituiert oder 1-3fach substituiert ist mit F, Cl, Br, (C₁-C₄)-Alkyl oder (C₁-C₄)-Alkoxy oder
ein R'''-(C=O)-Rest, wobei R''' Wasserstoff oder (C₁-C₄)-Alkyl bedeutet.

Ganz besonders bevorzugt sind 2 Homopolymere, die wie nachstehend beschrieben, hergestellt wurden.

In den folgenden Beispielen werden Gallensäuremethylester der allgemeinen Formel VI verwendet.

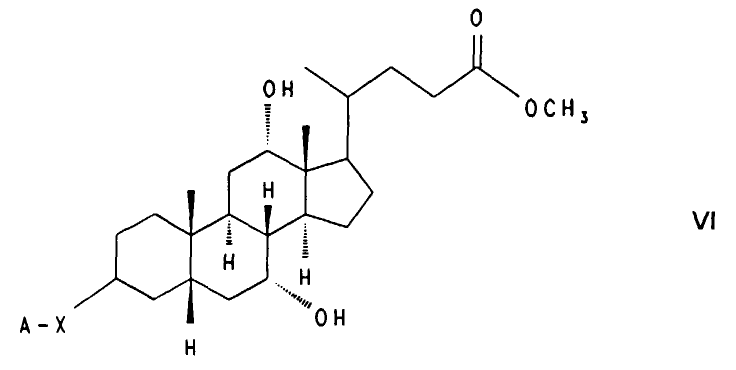

Die Gruppe A-X ist jeweils in den Beispielen definiert.

Für die Dialyse wurde Dialyseschlauch der Firma Spectrum Medical Industries, INC. mit der Bezeichnung Spectra/Por Nr. 3 mit einer Ausschlußgrenze von 3500 g/mol eingesetzt.

Die Bestimmung der gewichtsmittleren Molekulargewichte erfolgte mittels GPC im Vergleich zu Polystyrolstandards.
- Chromatograph:: ALC/GPC 244 Waters Chromatographie
- Säulensatz;: 4 Ultrastyragel Säulen
- Lösemittel:: THF
- Durchfluß:: 1 ml/min
- Probenmenge:: 0,4 ml Probenlösung mit c = 0,2 g/dl
- Detektor:: RI + 4X

### Verbindung 1

In einem Reaktionsgefäß werden unter Stickstoff 1110 mg eines Gallensäuremethylesters, wobei A-X = H₂C=CH-(C=O)-NH-(CH₂)₆-O- bedeutet, in 8 ml Tetrahydrofuran gelöst und mit 150 mg 75 %igem Dibenzoylperoxid, gelöst in 0,75 ml Toluol, versetzt. Die Reaktionsmischung wird unter Rühren für 18 Stunden auf 75°C erhitzt. Anschließend verdünnt man die Reaktionsmischung mit 10 ml THF und versetzt mit 0,5 ml 20 %iger wäßriger Natronlauge. Nach ca. 10 Minuten wird die auftretende Trübung der Reaktionsmischung durch Zusatz von Wasser in Lösung gebracht. Dieser Vorgang wird so oft wiederholt, bis keine Trübung der Mischung mehr auftritt. Anschließend wird die Reaktionsmischung mit 30 ml Wasser verdünnt, 24 Stunden gegen entsalztes Wasser dialysiert (cut off: 3500 g/mol) und gefriergetrocknet.

Gewichtsmittleres Molekulargewicht der unverseiften Substanz:
M_{w} = 10 000 g/mol (ermittelt mit GPC).

### Verbindung 2

In einem Reaktionsgefäß werden unter Stickstoff 386,8 mg eines Gallensäuremethylesters, wobei A-X = H₂C=CH-(C=O)-NH-(CH₂)₂-O- bedeutet, in 2,78 ml Tetrahydrofuran gelöst und mit 52 mg 75 %igem Dibenzoylperoxid, gelöst in 0,5 ml Toluol, versetzt. Die Reaktionsmischung wird unter Rühren für 18 Stunden auf 75°C erhitzt. Anschließend verdünnt man die Reaktionsmischung mit 10 ml THF und versetzt mit 0,5 ml 20 %iger wäßriger Natronlauge. Nach ca. 10 Minuten wird die auftretende Trübung der Reaktionsmischung durch Zusatz von Wasser in Lösung gebracht. Dieser Vorgang wird so oft wiederholt, bis keine Trübung der Mischung mehr auftritt. Anschließend wird die Reaktionsmischung mit 30 ml Wasser verdünnt, 24 Stunden gegen entsalztes Wasser dialysiert (cut off: 3500 g/mol) und gefriergetrocknet.

Gewichtsmittleres Molekulargewicht der unverseiften Substanz:
M_{w} = 11 000 g/mol (ermittelt mit GPC)

### 1. Herstellung der Antikörper

Das entsprechende Homopolymer, wurde zunächst mit 3 mg in kompletten Freund'schen Adjuvanz homogenisiert und anschließend für die Immunisierung verwendet. Die Darreichung erfolgte 8 mal in einem Zeitraum von 16 Wochen. Die jeweils injizierte Menge lag im Bereich von jeweils 0.001g - 10 mg des Homopolymer, suspendiert in inklompletten Freund'schen Adjuvanz und wurde vorzugsweise intramuskulär in ein geeignetes Wirbeltier, z.B. Kaninchen, Schwein, Schaf, Ziege, Huhn etc. appliziert. 10 Tage nach der jeweiligen Injektion wurde eine Serumprobe gewonnen und mit einem geeigneten Testverfahren, z.B. mit dem Dot-Blot-Verfahren, mit ELISA-Methodik oder mit Hilfe eines RIA-Verfahrens auf das Vorhandensein von den erfindungsgemäßen Antikörpern untersucht (siehe auch Punkt 2). Bei Hühnern ist es möglich 10 Tage nach der Injektion die Eier zu sammeln und aus ihnen die Antikörper unter Verwendung des EGGstract® Chicken-IgY-Purification System der Firma Promega zu erhalten.

### 2. Nachweis von Antikörpern gegen das Cholsäure-Homopolymer im Serum eines Spendertieres im Dot-Blot Verfahren

Verschiedene kommerziell erhältliche Gallensäuren (Firma Sigma) oder das Gallensäure-Homopolymer werden in einer Konzentration von 1 mg/ml in IEF-Puffer (0.25M Tris-Base, 2 M Glycin, 0.02% Natriumdodecylsulfat, 0.25% Nonidet® NP40 (Firma Sigma), 25 mg/25 ml Dithiotreitol, 37 mg/25 ml EDTA) gelöst Diese Stammlösungen werden in verschiedenen Verdünnungen (1:10-1:10.000) eingesetzt und jeweils 10 µl auf eine mit Methanol/Wasser aktivierte Immobilon®-Membran (Firma Millipore) aufgebracht. Anschließend wird die Membran bei 60°C im Trockenschrank getrocknet, um die Antigene auf der Membran zu fixieren. Die Membran wird wiederum durch Auflegen, mit der nicht mit Antigen beschichteten Seite, auf Methanol/Wasser aktiviert. Das Blockieren unbeschichteter Membranbereiche zur Vermeidung unspezifischer Antikörperbindung erfolgt, indem die Membran bei 4°C mit mit 5% Low fat dry milk (LFDM) in TBS (Tris buffered saline) über Nacht inkubiert wird. Anschließend wird 3 x 10 min mit 0.1% LFDM/TBS gewaschen und die Membran für 120 min mit der in TBS verdünnten Antiserumlösung (Verdünnungen bis 1:1000) inkubiert. Wiederum wird 3 x 10 min gewaschen und mit einem geeigneten zweiten Antikörper in Literatur bekannter Art inkubiert, der z.B. radioaktiv-, Fluoureszenz-, Chemilumineszenz- oder Enzymmarkiert sein kann. Stammt der erste Antikörper, welcher gegen ein bestimmtes Antigen generiert wurde aus dem Schaf, so nimmt man zum Nachweis, ob eine Antigen-Antikörper Reaktion erfolgt ist, beispielsweise einen Anti-Schaf-Peroxidase-Antikörper (Verdünnung 1:1000 in 0.1% LFDM/TBS). Als Farbreagenz der Peroxidasereaktion kann dann z.B. 10 mg Diaminobenzidin in 15 ml 0.1M Tris/HCL-Puffer pH 7.4 gelöst und die Nachweisreaktion durch Zugabe von 12 µl 30%igem H₂O₂ gestartet werden.

Im Falle der Erkennung eines Gallensäure-Homopolymers, konnte bei einer (Antiserumverdünnung 1 : 200 bis zu 1: 5000) das Antigen konzentrationsabhängig nachgewiesen werden (Verwendung einer 1 mg/ml Stammlösung). Der Nachweis freier Gallensäuren ist in geringen Verdünnungen nur qualitativ zu erzielen, da die unterschiedlichen Gallensäuren unterschiedliche Hydrophobizität aufweisen und dadurch die Haftung z.B. für Chenodeoxycholsäure an der Membran nicht identisch ist mit z.B. Taurocholsäure bzw. die vielen unterschiedlichen Gallensäuren an der Immobilon-Membran nurmehr mit unterschiedlicher Affinität haften.

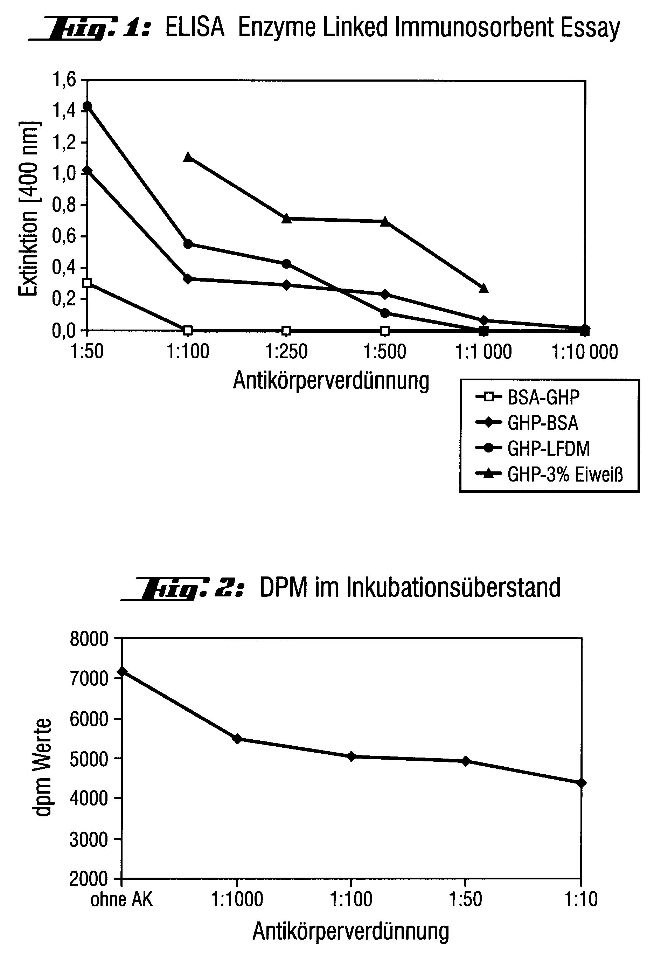

### 2.1. ELISA (Enzyme Linked Immunosorbent Assay) zur Bestimmung von Antikörpern gegen das Cholsäure-Homopolymer (Figur 1)

Die Antikörper im gewonnenen Vollserum aus den immunisierten Tieren werden durch Ammoniumsulfatpräzipitation aufgereinigt. Dazu wird gemäß der Anleitung aus 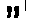 Biochemische Arbeitsmethoden" (T.G. Cooper, W. de Gruyter Verlag) das Serum mit einer gesättigten Ammoniumsulfatlösung durch sehr langsames Einrühren bei 4°C auf einen 25%igen (NH₄)₂SO₄-Sättigungsgrad eingestellt. Nach 6 Stunden rühren bei 4°C wird der Niederschlag mit einer Zentrifugation bei 3.000 x g abgetrennt. Anschließend wird mit der gesättigten (NH₄)₂SO₄ -Lösung auf einen 50%igen (NH₄)₂SO₄-Sättigungsgrad eingestellt. Wiederum wird für 6 Stunden bei 4 °C gerührt und der die Antikörper enthaltende Niederschlag durch eine 3.000 x g Zentrifugation gewonnen. Das Präzipitat wird in TBS aufgenommen und für 3 x 12 h gegen TBS-0.05% Natriumazid pH 7.5 bei 4°C dialysert.

Da Gallensäuren ebenfalls eine hohe Albuminbindung aufweisen, wurden parallel verschiedene Blockierungslösungen ausgetestet, um einerseits die unspezifische Bindungen sowie Interferenzen der Blockierungslösung beurteilen zu können.
- Lösung 1:: 0.1M Natriumcarbonat pH 9.6
0.02% Natriumazid
- Lösung 2:: 0.015% Tween® 20 (Firma Sigma) in TBS pH 7.4
- Lösung 3:: 50 mM Glycin, pH 9.6
0.5 mM MgCl₂
direkt vor Inkubation wird 1 mg/ml p-Nitrophenylphosphat gelöst.

### Ansatz a:

3% BSA (Bovine Serum Albumin) in Lösung 1 wurde über Nacht bei 4°C in 96-Loch- Plastikplatten inkubiert, anschließend wurde 3 x mit Lösung 2 gewaschen. Danach wurde das Cholsäure-Homopolymer in einer Konzentration von 0.1 mg/ml in Lösung 1 für 3 Stunden bei RT inkubiert. Bevor das Anti-Cholsäure-Homopolymer-Antiserum in verschiedenen Verdünnungen in Lösung 1 für 3 Stunden inkubiert wurde, wurde 3 mal mit Lösung 2 gewaschen. Anschließend wurde unspezifisch gebundenes Antiserum durch 3 maliges Waschen mit Lösung 2 entfernt. Der Nachweis erfolgte mit einem Anti-Schaf IgG-Antikörper (erzeugt in Esel oder Ziege) der mit einer alkalischen Peroxidase gekoppelt ist.

### Ansatz b:

0.1 mg/ml Cholsäure-Homopolymer in Lösung 1 wurde über Nacht bei 4°C in 96-Loch-Plastikplatten inkubiert, anschließend wurde 3 x mit Lösung 2 gewaschen. Nachfolgend wurde mit 3% BSA in Lösung 1 für 3 Stunden bei RT inkubiert. Bevor das Anti-Cholsäure-Homopolymer-Antiserum in verschiedenen Verdünnungen in Lösung 1 für 3 Stunden inkubiert wurde, wurde 3 mal mit Lösung 2 gewaschen. Anschließend wurde unspezifisch gebundenes Antiserum durch 3 maliges Waschen mit Lösung 2 entfernt. Der Nachweis erfolgte mit einem Anti-Schaf-IgG-Antikörper (erzeugt in Esel oder Ziege) der mit einer alkalischen Peroxidase gekoppelt ist.

### Ansatz c:

0.1 mg/ml Cholsäure-Homopolymer in Lösung 1 wurde über Nacht bei 4°C in 96-Loch-Plastikplatten inkubiert, anschließend wurde 3 x mit Lösung 2 gewaschen. Nachfolgend wurde mit 3% Low fat dry milk in Lösung 1 für 3 Stunden bei RT inkubiert. Bevor das Anti-Cholsäure-Homopolymer-Antiserum in verschiedenen Verdünnungen in Lösung 1 für 3 Stunden inkubiert wurde, wurde 3 mal mit Lösung 2 gewaschen. Anschließend wurde unspezifisch gebundenes Antiserum durch 3 maliges Waschen mit Lösung 2 entfernt. Der Nachweis erfolgte wie in a) beschrieben.

### Ansatz d:

0.1 mg/ml Cholsäure-Homopolymer in Lösung 1 wurde über Nacht bei 4°C in Elisa-platten inkubiert, anschließend wurde 3 x mit Lösung 2 gewaschen. Nachfolgend wurde mit 3% Hühnereiweiß (Firma Sigma) in Lösung 1 für 3 Stunden bei RT inkubiert. Bevor das Anti-Cholsäure-Homopolymer-Antiserum in verschiedenen Verdünnungen in Lösung 1 für 3 Stunden inkubiert wurde, wurde 3 mal mit Lösung 2 gewaschen. Anschließend wurde unspezifisch gebundenes Antiserum durch 3 maliges Waschen mit Löung 2 entfernt. Der Nachweis erfolgte wie in a) beschreiben.

Reaktion mit dem Alkalische Peroxidase markierten Anti-Schaf-IgG-Antikörper Der kommerziell erhältliche Antikörper wurde in einer Verdünnung von 1:1000 in Lösung 1 für 2 Stunden inkubiert, nach 3 maligem Waschen mit Lösung 1 (ohne Natriumazid) wurde die Nachweisreaktion durch Zugabe von Lösung 3 gestartet und die Extinktion bei 400 nm nach 15 Minuten Reaktionsdauer im ELISA-Reader bestimmt (siehe Figur 1).

In Figur 1 ist als O-Wert die entsprechende Antikörper-verdünnung ohne Inkubation mit dem Gallensäure-Homopolymer (GHP) abgezogen.

### 3. Bestimmung von Gallensäuren mit dem Antiserum gegen Gallensäure-Homopolymere (Figur 2)

1.5 ml des erfindungsgemäßen Antiserums, z.B. aus Schaf wird mit 75 mg Aktivkohle für 15 Minuten gerührt, um im Serum vorhandende Gallensäuren zu entfernen. Die Aktivkohle wird anschließend durch 20 min Zentrifugation bei 9.000 x g und durch Filtration durch einen 0.45 µm Filter abgetrennt. Das Serum wird weiterhin 1:2 mit 0.01M Kaliumphosphatpuffer pH 7.4 (KPP-Puffer) verdünnt.

Antikörper im Antiserum werden mit dem Protein-G Kit der Firma Pierce gereinigt und eine Verdünnungsreihe mit KPP-Puffer bis zu 1: 10.000 hergestellt.

100 µl einer [¹⁴C]-TCA-Lösung ( 8000 dpm) wird mit 100 µl der verschieden verdünnten Antikörperlösungen zusammengegeben und mit KPP-Puffer auf 1000 µl aufgefüllt. Die Probe wird bei 42°C für 60 Minuten, und anschließend bei 4°C für 45 Minuten inkubiert. Anschließend wird 0.5 ml einer 37.5% Polyethylenglykol-Lösung in KPP zugegeben und für weitere 10 Minuten inkubiert. Danach erfolgt eine Präzipitation bei 1200 x g bei 4°. Der Überstand wird abgenommen und die nicht nach Antikörperbindung ausgefällte [¹⁴C]-TCA-Menge im Szintillationszähler nach Zugabe von 10 ml Szintillationsfüssigkeit ausgezählt (siehe Figur 2).

Mit dem erzeugten Antikörper läßt sich das Gallensäure-Homopolymer im ELISA konzentrationsabhängig nachweisen und identifizieren. Daß dieser polyklonale Antikörper selbst zum Nachweis stark verdünnter Gallensäureproben eingesetzt werden kann, da auch in starker Verdünnung die Antigen-Antikörperreaktion erfolgt, ist durch Verwendung von ¹⁴C-TCA gezeigt. Somit eignet sich dieser Antikörper auch zur Analyse von Serumproben mit geringer Gallensäuregehalt.

Die verwendeten Abkürzungen bedeuten:
- IEF: = Isoelektrische Fokussierung
- KPP: = Kaliumphosphatpuffer
- TCA: = Taurocholsäure
- EDTA: = Ethylendinitrilotetraessigsäure-Dinatriumsalz
- GPC: = Gel Permeations Chromatographie
- TBS: = Tris Buffered Saline
- LFDM: = Low Fat Dry Milk
- BSA: = Bovine Serum Albumin
- RIA: = Radio Immuno Assay
- ELISA: = Enzyme Linked Immonosorbent Assay

## Patentansprüche

1. Antikörper mit Spezifität zu Gallensäuren, herstellbar durch Immunisierung einer geeigneten Wirbeltierart mit einem Homopolymer, welches hergestellt wird durch Polymerisation von monomeren Gallensäuren der Formel I
G - X - A (I)
in der
G ein Gallensäurerest oder -derivat,
X eine Brückengruppe und
A eine polymersierbare, ethylenisch ungesättigte Gruppe bedeuteten.

2. Antikörper nach Anspruch 1, dadurch gekennzeichnet, daß darin bedeuten
G eine freie Gallensäure, ihr Alkali- oder Erdalkalisalz oder eine am Ring D veresterte Gallensäure, die über ihren Ring A verbunden ist mit der Brückengruppe Gruppe X
X eine Brückengruppe der Formel II
(Y)ₒ - (Z)ₚ (II),
Y -O-, -NR'-, -O-(C=O)-, -NR'-(C=O)-, wobei Y benachbart zu G steht,
Z (C₁-C₁₂)-Alkylen oder (C₇-C₁₃)-Aralkylen, wobei einzelne Methylengruppen in der Alkylenkette des Alkylen- oder Aralkylenrestes durch Gruppen wie -O-, -NR'-, -NR'-(C=O)-, -O-(C=O)- oder -NR'-(C=O)-NR''-, ersetzt sein können,
o, p unabhängig voneinander null oder 1, wobei o und p nicht gleichzeitig null sind,
A eine ethylenisch ungesättigte Gruppe der Formel 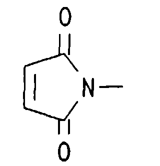 oder 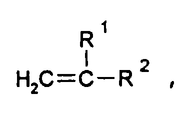
R¹ Wasserstoff oder CH₃,
R² -NR'-(C=O)-, -O-(C=O)-, -O-, -NR'- oder eine Einfachbindung, wobei die Carbonylgruppen benachbart zur C-C-Doppelbindung stehen,
R',R'' Wasserstoff oder (C₁-C₆)-Alkylen.

3. Antikörper nach einem der Ansprüche 1 oder 2, dadurch gekennzeichnet, daß darin bedeuten
G 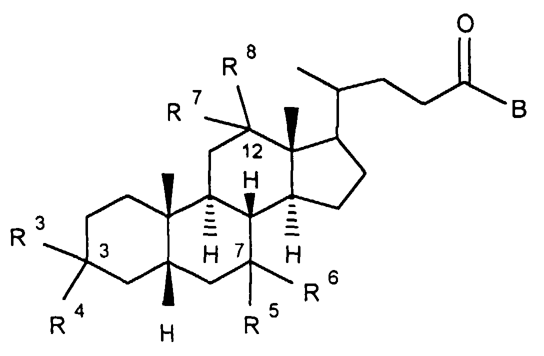
R³ bis R⁸ unabhängig voneinander Wasserstoff, OH, NH₂ oder eine mit einer OH-Schutzgruppe geschützte OH-Gruppe, worin einer der Reste R³ oder R⁴ eine Bindung zur Gruppe X bedeutet,
B -OH, -O-Alkali, -O-Erdalkali, -O-(C₁ -C₁₂)-Alkyl, -O-Allyl oder -O-Benzyl, wobei Alkyl sowohl n-Alkyl wie iso-Alkyl bedeutet und wobei die gebildete Estergruppe 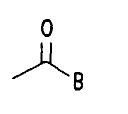 sowohl sauer wie auch basisch verseifbare Ester darstellt,
Y -O-, NR'-, -O-(C=O)-, -NR'-(C=O)-,
Z (C₁ -C₁₂)-Alkylen, (C₇-C₁₃)-Aralkylen, wobei einzelne Methylengruppen in der Alkylenkette durch die Gruppen -O-, -NR'-, -NR'-(C=O)-, -O-(C=O)-, -NR'-(C=O)-NR''- ersetzt sein können,
o, p unabhängig voneinander null oder 1 bedeuten, wobei o und p nicht gleichzeitig null sind,
A 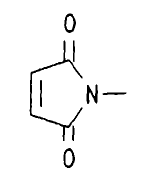 oder 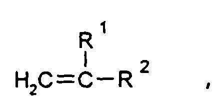
R¹ Wasserstoff oder CH₃,
R² -NR'-(C=O)-, -NR'- oder eine Einfachbindung,
R' Wasserstoff oder (C₁-C₆)-Alkylen.

4. Antikörper nach einem oder mehreren der Ansprüche 1 bis 3, dadurch gekennzeichnet, daß das Homopolymer durch 18-stündige Umsetzung bei 75°C von 1110 mg des Gallensäuremethylesters der Formel 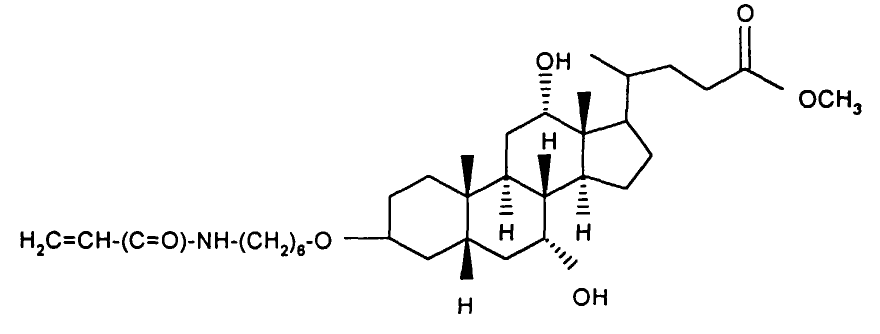 gelöst in 8 ml Tetrahydrofuran mit 150 mg 75 %igem Dibenzoylperoxid, gelöst in 0,75 ml Toluol unter Stickstoffatomosphäre herstellt wird.

5. Antikörper nach einem oder mehreren der Ansprüche 1 bis 3, dadurch gekennzeichnet, daß das Homopolymer durch 18-stündige Umsetzung bei 75°C von 386,8 mg des Gallensäuremethylesters der Formel 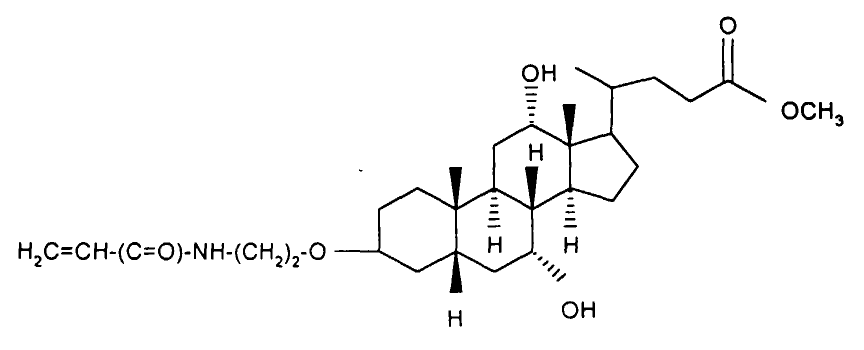 gelöst in 2,78 ml Tetrahydrofuran mit 52 mg 75 %igem Dibenzoylperoxid, gelöst in 0,5 ml Toluol unter Stickstoffatomosphäre herstellt wird.

6. Verfahren zur Herstellung eines Antikörpers gemäß einem oder mehreren der Ansprüche 1 bis 5, dadurch gekennzeichnet, daß Wirbeltiere mit dem Homopolymer, wie in Ansprüchen 1 bis 5 beschrieben, immunisiert werden und das Serum mit den erfindungsgemäßen Antikörpern gewonnen wird.

7. Verwendung der Antikörper gemäß einem oder mehreren der Ansprüche 1 bis 5 in einem Immunoassay zur Bestimmung von Gallensäuren in biologischen Matrices.

8. Immunoassay-Kit zur Bestimmung von Gallensäuren in biologischen Matrices, enthaltend einen Antikörper nach einem oder mehreren der Ansprüche 1 bis 5.
